(19) Europäisches Patentamt
European Patent Office
Office européen des brevets

(11) Veröffentlichungsnummer: **0 022 491**
**B1**

(12) # EUROPÄISCHE PATENTSCHRIFT

(45) Veröffentlichungstag der Patentschrift: **06.04.83**

(51) Int. Cl.³: **C 07 D 413/10**, C 08 K 5/35, C 11 D 3/42, D 06 L 3/12

(21) Anmeldenummer: **80103581.7**

(22) Anmeldetag: **25.06.80**

(54) **Neue Stilbenverbindungen, Verfahren zu deren Herstellung und deren Verwendung als optische Aufheller.**

(30) Priorität: **29.06.79 DE 2926234**

(43) Veröffentlichungstag der Anmeldung: **21.01.81 Patentblatt 81/3**

(45) Bekanntmachung des Hinweises auf die Patenterteilung: **06.04.83 Patentblatt 83/14**

(84) Benannte Vertragsstaaten: **AT BE CH DE FR GB IT LI NL SE**

(56) Entgegenhaltungen:
DE-A-2 535 612
DE-A-2 712 409
DE-A-2 745 998

„Helvetica Chimica Acta", Bd. 57, Nrn. 7-8, Fasc. 1, (1974) Basel, A.E. Siegrist „Anil-Synthese. 9. Mitteilung. Über die Darstellung von in 4'-Stellung heterocyclisch *substituierten* 4-([2H]-Arenotriazol-2-yl)-stilbenen", S. 81 bis 103
„Chemical Abstracts", Bd. 91, 1979 Columbus, Ohio, USA Chemical Substance Index, S. 1035 CS

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt/Main 80 (DE)**

(72) Erfinder: **Eckes, Helmut, Dr., Feldbergblick 5, D-6239 Eppstein/Taunus (DE)**
Erfinder: **Erckel, Rüdiger, Dr., Staufenstrasse 16, D-6239 Eppstein/Taunus (DE)**
Erfinder: **Martini, Thomas, Dr., Am Schellberg 42, D-6232 Bad Soden am Taunus (DE)**
Erfinder: **Rösch, Günter, Hohlweg 17, D-6232 Bad Soden am Taunus (DE)**

**EP 0 022 491 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue Stilbenverbindungen, Verfahren zu deren Herstellung und deren Verwendung als optische Aufheller

Aus „Helv. Chim. Acta", vol. 57 (1974), S. 88 und 92 sind bereits Benzotriazolyl- bzw. Naphthotriazolyl-3-phenyl-1,2,4-oxadiazol-5-ylstilben Verbindungen als optische Aufheller bekannt, die in der 3-Penylgruppe weiter durch Methyl oder Chlor substituiert sein können. Es wurde nun gefunden, dass man die Aufhellwirkung solcher Verbindungen noch verbessern kann, wenn man diese Arylsubstituenten am Oxdiazolylring durch Alkylsubstituenten ersetzt.

Gegenstand der vorliegenden Anmeldung sind 4-Triazolylstilbenverbindungen der allgemeinen Formel 1

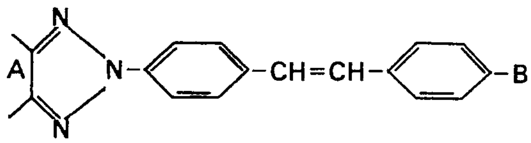 (1)

worin A einen gegebenenfalls durch nichtchromophore Substituenten substituierten Benzol- oder Naphthalinring, B eine Gruppe der Formeln

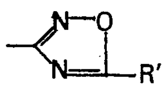 oder 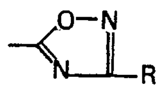

und R und R' ($C_1$ bis $C_6$)-Alkyl, ($C_1$ bis $C_6$)-Chloralkyl, ($C_1$ bis $C_4$)-Alkyloxy-($C_1$ bis $C_4$)-Alkyl, Hydroxy-($C_1$ bis $C_4$)-Alkyl oder eine Gruppe der Formel $-(CH_2CH_2O)_n-R^1$ bedeuten, wobei n 2 oder 3 und $R^1$ Wasserstoff oder ($C_1$ bis $C_4$)-Alkyl ist.

Besonders bevorzugt sind Verbindungen der allgemeinen Formel 1, wo A eine Gruppe der Formel

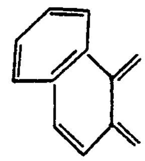

und B für eine Gruppe der Formel

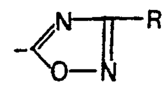

steht, in der R ($C_1$ bis $C_4$)-Alkyl, ($C_1$ bis $C_4$)-Chloralkyl, Hydroxy-($C_1$ bis $C_4$)-Alkyl und ($C_1$ bis $C_4$)-Alkoxyalkyl bedeutet.

Im einzelnen kommen für R und R' folgende Gruppen in Frage: Methyl, Äthyl, n- oder i-Propyl, n- oder i-Butyl, Pentyl, Hexyl oder die sich davon ableitenden Chloralkyl-, Hydroxyalkyl-, Methoxyalkyl-, Äthoxyalkyl-, Propoxyalkyl- bzw. Butoxyalkylgruppen. Ferner Gruppen der Formel $-(CH_2CH_2O)_n-R^1$, wo n 2 oder 3 und $R^1$ ein Wasserstoffatom, eine Methyl-, Äthyl-, Propyl- oder Butylgruppe ist.

Beispiele hierfür sind die Reste der Formeln

$-(CH_2CH_2O)_2CH_3$, $-(CH_2CH_2O)_2C_2H_5$,
$-(CH_2CH_2O)_2C_4H_9$, $-(CH_2CH_2O)_3C_2H_5$.

Ganz besonders zu nennen sind folgende Verbindungen

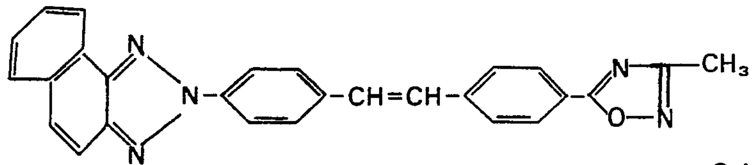 (4)

$-C_2H_5$ (5)

$-CH_2-CH_2OH$ (6)

$-CH_2-CH_2-OCH_3$ (7)

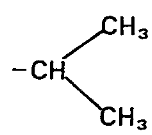 (8)

Gegenstand der Erfindung ist ferner ein Verfahren zur Herstellung der Verbindungen der Formel 1, dadurch gekennzeichnet, dass man eine Verbindung der allgemeinen Formel 9

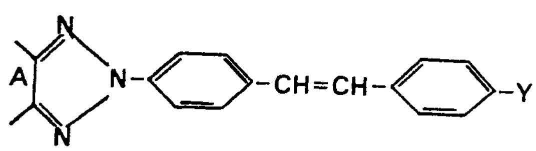 (9)

mit einer Verbindung der allgemeinen Formel 10

$$R-Z \quad (10)$$

umsetzt, wobei R die zuvor angegebenen Bedeutungen hat, und Y eine Gruppe der Formel 11

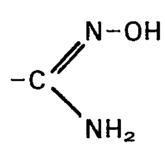 (11)

bedeutet, wobei Z gleichzeitig eine Gruppe der Formel 12

$$-COCl \quad (12)$$

darstellt oder Y bedeutet eine Gruppe der Formel 12 und Z ist gleichzeitig eine Gruppe der Formel 11.

Im ersten Fall erhält man solche Verbindungen der Formel 1, die eine 1,2,4-Dioxazolyl-3-gruppe enthalten und im zweiten Fall enthalten die Verbindungen die 1,2,4-Dioxazolyl-5-gruppe. Die Umsetzung erfolgt vorzugsweise in Gegenwart eines säurebindenden Mittels in einem inerten Lösungsmittel bei Temperaturen von 20°-200° C. Als Lösungsmittel für die Reaktion eignen sich z.B. Chlorbenzol, Di- oder Trichlorbenzol und insbesondere Dimethylformamid, N-Methylpyrrolidon, Dimethylsulfoxid oder Nitrobenzol. Als säurebindende Mittel sind beispielsweise Natriumcarbonat, Caliumcarbonat, Calciumcarbonat, Triäthylamin oder Äthyldiisopropylamin verwendbar.

Die Verbindungen der Formel 9, worin Y eine Gruppe der Formel 12 bedeutet, sind bekannt und z.B. in den DE-OS 2539461 und DE-OS 2539537 beschrieben, bzw. können analog zu bekannten Verfahren hergestellt werden. Hieraus lassen sich nach allgemein bekannten Verfahren, z.B. durch Aminolyse und anschliessende Dehydratisierung, die Verbindung der allgemeinen Formel 9, in der Y die Nitrilgruppe bedeutet, herstellen.

Die Verbindungen der Formel 9, wobei Y eine Gruppe der Formel 11 bedeutet, erhält man aus den oben beschriebenen Nitrilen in bekannter Weise durch Umsetzung mit Hydroxylamin, vorzugsweise in Alkoholen oder N-Methylpyrrolidon.

Die vorstehend definierten neuen Verbindungen zeigen in gelöstem oder feinverteiltem Zustand eine mehr oder weniger ausgeprägte Fluoreszenz. Sie eignen sich deshalb zum optischen Aufhellen der verschiedensten synthetischen, halbsynthetischen oder natürlichen organischen Materialien enthalten.

Hierfür seien beispielsweise, ohne dass durch die nachfolgende Übersicht irgendeine Beschränkung hierauf ausgedrückt werden soll, die folgenden Gruppen von organischen Materialien, soweit eine optische Aufhellung derselben in Betracht kommt, genannt:

I. Synthetische organische hochmolekulare Materialien:

a) Polymerisationsprodukte auf Basis mindestens eine polymerisierbare Kohlenstoff-Kohlenstoff-Doppelbindung enthaltender organischer Verbindungen, d.h. deren Homo- oder Copolymerisate sowie deren Nachbehandlungsprodukte wie beispielsweise Vernetzungs-, Pfropfungs- oder Abbauprodukte, Polymerisat-Verschnitte oder durch Modifizierung reaktionsfähiger Gruppen erhaltene Produkte, beispielsweise Polymerisate auf Basis von α,β-ungesättigten Carbonsäuren oder Derivaten solcher Carbonsäuren, insbesondere von Acrylverbindungen (wie z.B. Acrylestern, Acrylsäure, Acrylnitril, Acrylamiden und deren Derivaten oder deren Methacryl-Analoga), von Olefin-Kohlenwasserstoffen (wie z.B. Äthyle, Propylen, Styrole oder Diene, ferner sogenannte ABS-Polymerisate), Polymerisate auf Basis von Vinyl- und Vinyliden- Verbindungen (wie z.B. Vinylchlorid, Vinylalkohol, Vinylidenchlorid),

b) Polymerisationsprodukte, die durch Ringöffnung erhältlich sind, z.B. Polyamide vom Polycaprolactam-Typ, ferner Polymere, die sowohl über Polyaddition als auch Polykondensation erhältlich sind, wie Polyäther oder Polyacetale,

c) Polykondensationsprodukte oder Vorkondensate auf Basis bi- oder polyfunktioneller Verbindungen mit kondensationsfähigen Gruppen, deren Homo- und Mischkondensationsprodukte sowie Produkte der Nachbehandlung, wie beispielsweise Polyester, insbesondere gesättigte (z.B. Äthylenglykolterephthalsäure-Polyester) oder ungesättigte (z.B. Maleinsäure-Dialkohol-Polykondensate sowie deren Vernetzungsprodukte mit anpolymerisierbaren Vinylmonomeren), unverzweigte sowie verzweigte (auch auf Basis höherwertiger Alkohole, wie z.B. Alkydharze) Polyester, Polyamide (z.B. Hexamethylendiamin-adipat), Maleinharze, Melaminharze, deren Vorkondensate und Analoge, Polycarbonate, Silikone,

d) Polyadditionsprodukte wie Polyurethane (vernetzt und unvernetzt), Epoxidharze.

II. Halbsynthetische organische Materialien, z.B. Celluloseester verschiedener Veresterungsgrade (sogenanntes 2½-Acetat, Triacetat) oder Celluloseäther, regenerierte Cellulose (Viskose, Kupferammoniak-Cellulose) oder deren Nachbehandlungsprodukte, Casein-Kunststoffe.

III. Natürliche organische Materialien animalischen oder vegetabilischen Ursprungs, beispielsweise auf Basis von Cellulose oder Proteinen wie Baumwolle, Wolle, Leinen, Seiden, natürliche Lackharze, Stärke, Casein.

Die optisch aufzuhellenden organischen Materialien können den verschiedenartigsten Verarbeitungszuständen (Rohstoffe, Halbfabrikate, oder Fertigfabrikate) angehören. Sie können andererseits in Form der verschiedenartigsten geformten Gebilden vorliegen, d.h. beispielsweise als vorwiegend dreidimensional ausgedehnte Körper wie Platten, Profile, Spritzgussförmlinge, verschiedenartige Werkstücke, Schnitzel, Granulate oder Schaumstoffe, ferner als vorwiegend zweidimensional ausgebildete Körper wie Filme, Folien, Lacke, Überzüge, Imprägnierungen und Beschichtungen oder als vorwiegend eindimensional ausgebildete Körper wie Fäden, Fasern, Flocken, Drähte. Die besagten Materialien können andererseits auch in ungeformten Zuständen in den verschiedenartigsten homogenen oder inhomogenen Verteilungsformen, wie z.B. als Pulver, Lösungen, Emulsionen, Dispersionen, Latices, Pasten oder Wachse vorliegen.

Fasermaterialien können beispielsweise als endlose Fäden (verstreckt oder unverstreckt), Stapelfasern, Flocken, Strangware, textile Fäden, Garne, Zwirne, Faservliese, Filze, Watten, Beflokkungs-Gebilde oder als textile Gewebe oder textile Verbundstoffe, Gewirke sowie als Papiere, Pappen oder Papiermassen vorliegen.

Den erfindungsgemäss anzuwendenden Verbindungen kommt u.a. Bedeutung für die Behandlung von textilen organischen Materialien, insbesondere textilen Geweben, zu. Sofern Fasern, welche als Stapelfasern oder Endlosfäden, in Form von Strängen, Geweben, Gewirken, Vliesen, beflockten Substraten oder Verbindstoffen vorliegen

können, erfindungsgemäss optisch aufzuhellen sind, geschieht dies mit Vorteil in wässerigem Medium, worin die betreffenden Verbindungen in feinverteilter Form (Suspensionen, sogenannten Mikrodispersionen, gegebenenfalls Lösungen) vorliegen. Gegebenenfalls können bei der Behandlung Dispergier-, Stabilisier-, Netz- und weitere Hilfsmittel zugesetzt werden.

In Abhängigkeit vom verwendeten Aufheller-Verbindungstyp kann es sich als vorteilhaft erweisen, in neutralem oder alkalischem oder saurem Bade zu arbeiten. Die Behandlung wird üblicherweise bei Temperaturen von etwa 20 bis 140° C, beispielsweise bei Siedetemperatur des Bades oder in deren Nähe (etwa 90° C) durchgeführt. Für die erfindungsgemässe Veredelung textiler Substrate kommen auch Lösungen oder Emulsionen in organischen Lösungsmitteln in Betracht, wie dies in der Färbereipraxis in der sogenannten Lösungsmittelfärberei (Foulard-Thermofixierapplikation, Ausziehfärbeverfahren in Färbemaschinen) praktiziert wird.

Die neuen optischen Aufhellmittel gemäss vorliegender Erfindung können ferner den Materialien vor oder während deren Verformung zugesetzt bzw. einverleibt werden. So kann man sie beispielsweise bei der Herstellung von Filmen, Folien (z.B. Einwalzen in Polyvinylchlorid in der Hitze) oder Formkörpern der Pressmasse oder Spritzgussmasse beifügen.

Sofern die Formgebung voll- oder halbsynthetischer organischer Materialien durch Spinnverfahren bzw. über Spinnmassen erfolgt, können die optischen Aufheller nach folgenden Verfahren appliziert werden:

- Zugabe zu den Ausgangssubstanzen (z.B. Monomeren) oder Zwischenprodukten (z.B. Vorkondensaten, Präpolymeren), d.h. vor oder während der Polymerisation, Polykondensation oder Polyaddition,
- Aufpudern auf Polymerisationsschnitzel oder Granulate für Spinnmassen,
- Badfärbung von Polymerisatschnitzeln oder Granulaten für Spinnmassen,
- dossierte Zugabe zu Spinnschmelzen oder Spinnlösungen,
- Applikation auf Spinnkabel vor dem Verstrekken.

Die neuen optischen Aufhellmittel gemäss vorliegender Erfindung können beispielsweise auch in folgenden Anwendungsformen eingesetzt werden:

a) Mischung mit Farbstoffen (Nuancierung) oder Pigmenten (Farb- oder insbesondere z.B. Weisspigmenten) oder als Zusatz zu Färbebädern, Druck-, Ätz- oder Reservepasten. Ferner auch zur Nachbehandlung von Färbungen, Drucken oder Ätzdrucken,

b) in Mischungen mit sogenannten „Carriern", Netzmitteln, Weichmachern, Quellmitteln, Antioxydantien, Lichtschutzmitteln, Hitzestabilisatoren, chemischen Bleichnitteln (Chlorid-Bleiche, Bleichbäder-Zusätze),

c) in Mischungen mit Vernetzern, Appreturmitteln (z.B. Stärke oder synthetischen Appreturen) sowie in Kombination mit den verschiedensten Textilveredelungsverfahren, insbesondere Kunstharzausrüstungen (z.B. Knitterfest-Ausrüstungen wie „wash-and-wear", „permanent-press", „non-iron"), ferner Flammfest-, Weichgriff-Schmutzablöse („anti-soiling")-oder Antistatisch-Ausrüstungen oder antimokrobiellen Ausrüstungen,

d) Einarbeiten der optischen Aufhellmittel in polymeren Trägermaterialien (Polymerisations-, Polykondensations- oder Polyadditionsprodukte) in gelöster oder dispergierter Form zur Anwendung z.B. in Beschichtungs-, Imprägnier- oder Bindemitteln (Lösungen, Dispersionen, Emulsionen) für Textilien, Vliese, Papier, Leder,

e) als Zusätze zu sogenannten „master batches",

f) als Zusätze zu den verschiedensten industriellen Produkten, um dieselben marktfähiger zu machen (z.B. Aspektverbesserungen von Seifen, Waschmitteln, Pigmenten),

g) in Kombination mit anderen, optisch aufhellend wirkenden Substanzen,

h) in Spinnbadpräparationen, d.h. als Zusätze zu Spinnbädern, wie sie zur Gleitfähigkeitsverbesserung für die Weiterverarbeitung von Synthese-Fasern verwendet werden, oder aus einem speziellen Bad vor der Verstreckung der Faser,

i) als Szintillatoren, für verschiedene Zweck photographischer Art, wie z.B. für elektrophotographische Reinproduktion oder Supersensibilisierung,

j) je nach Substitution als Laser-Farbstoffe.

Wird das Aufhellverfahren mit Textil-Behandlungs- oder Veredlungsmethoden kombiniert, so kann die kombinierte Behandlung in vielen Fällen vorteilhafterweise mit Hilfe entsprechender beständiger Präparate erfolgen, welche die optisch aufhellenden Verbindungen in solcher Konzentration enthalten, dass der gewünschte Aufhelleffekt erreicht wird.

In gewissen Fällen werden die Aufheller durch eine Nachbehandlung zur vollen Wirkung gebracht. Diese kann beispielsweise eine chemische (z.B. Säure-Behandlung), eine thermische (z.B. Hitze) oder eine kombinierte chemisch/thermische Behandlung darstellen. So verfährt man beispielsweise bei der optischen Aufhellung einer Reihe von Fasersubstraten, z.B. von Polyesterfasern, mit den erfindungsgemässen Aufhellern zweckmässig in der Weise, dass man diese Fasern mit den wässerigen Dispersionen bei Temperaturen unter 75° C, z.B. bei Raumtemperatur, imprägniert und einer trockenen Wärmebehandlung bei Temperaturen über 100° C unterwirft, wobei es sich im allgemeinen empfiehlt, das Fasermaterial vorher noch bei mässig erhöhter Temperatur, z.B. bei mindestens 60° C bis etwa 130° C zu trocknen. Die Wärmebehandlung in trockenem Zustande erfolgt dann vorteilhaft durch Erwärmen in einer Trockenkammer, durch Bügeln im angegebenen Temperaturintervall oder auch durch Behandeln mit trockenem, überhitztem Wasserdampf. Die Trocknung und trockene Wärmebehandlung können auch unmittelbar nacheinander ausgeführt

oder in einem einzigen Arbeitsgang zusammengelegt werden.

Die Menge der erfindungsgemäss zu verwendenden neuen optischen Aufheller, bezogen auf das optisch aufzuhellende Material, kann in weiten Grenzen schwanken. Schon mit sehr geringen Mengen, in gewissen Fällen z.B. solchen von 0,001 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,005 und 2, vorzugsweise 0,1 bis 0,5 Gewichtsprozent von Interesse.

Aus verschiedenen Gründen ist es oft zweckmässig, die Aufheller nicht als solche, d.h. rein einzusetzen, sondern vermischt mit den verschiedensten Hilfs- und Coupiermitteln, wie z.B. wasserfreiem Natriumsulfat, Natriumsulfat-decahydrat, Natriumchlorid, Natriumcarbonat, Alkalimetallphosphaten, wie Natrium- oder Kaliumorthophosphat, Natrium- oder Kaliumpyrophosphat und Natrium- oder Kaliumtripolyphosphate oder Alkalimetallsilikaten.

Die neuen optischen Aufhellmittel eignen sich auch besonders als Zusätze für Waschbäder oder zu Gewerbe- und Haushaltswaschmitteln, wobei sie in verschiedener Weise zugesetzt werden können. Zu Waschbädern werden sie zweckmässig in Form ihrer Lösungen in Wasser oder organischen Lösungsmitteln oder auch in feiner Verteilung als wässerige Dispersionen zugegeben. Zu Haushalt- oder gewerblichen Waschmitteln werden sie vorteilhaft in irgendeiner Phase des Herstellungsprozesses der Waschmittel, z.B. der sogenannten „Slurry" vor dem Zerstäuben dem Waschpulver oder bei der Vorbereitung flüssiger Waschmittelkombinationen zugesetzt. Die Zugabe kann sowohl in Form einer Lösung oder Dispersion in Wasser oder anderen Lösungsmitteln als auch ohne Hilfsmittel als trockenes Aufhellpulver erfolgen. Man kann die Aufhellmittel beispielsweise mit den waschaktiven Substanzen vermischen, verkneten oder vermahlen und so dem fertigen Waschpulver zumischen. Sie können jedoch auch gelöst oder vordispergiert auf das fertige Waschmittel aufgesprüht werden.

Als Waschmittel kommen die bekannten Mischungen von Waschaktivsubstanzen wie beispielsweise Seife in Form von Schnitzeln und Pulver, Synthetika, lösliche Salze von Sulfonsäurehalbestern höherer Fettalkohole, höher und/oder mehrfach alkylsubstituierter Arylsulfonsäuren, Sulfocarbonsäureestern mittlerer bis höherer Alkohole, Fettsäureacylaminoalkyl-, oder -aminoacrylglycerinsulfonate, Phosphorsäureester von Fettalkoholen usw. in Frage. Als Aufbaustoffe, sogenannte „Builders", kommen z.B. Alkalipoly- und polymetaphosphate, Alkalipyrophosphate, Alkalisalze der Carboxymethylcellulose und andere „Soilredepositionsinhibitoren", ferner Alkalisilikate, Alkalicarbonate, Alkaliborate, Alkaliperborate, Nitrilotriessigsäure, Äthylendiaminotetraessigsäure, Schaumstabilisatoren wie Alkanolamide höherer Fettsäuren, in Betracht. Ferner können in den Waschmitteln beispielsweise enthalten sein:

Antistatische Mittel, rückfettende Hautschutzmittel wie Lanolin, Enzyme, Antimikrobika, Parfüme und Farbstoffe.

Die neuen optischen Aufheller haben den besonderen Vorteil, dass sie auch bei Gegenwart von Aktivchlorspendern, wie z.B. Hypochlorid, wirksam sind und ohne wesentliche Einbusse der Effekte in Waschbädern mit nichtionogenen Waschmitteln, wie z.B. Alkylphenolpolyglykoläthern, verwendet werden können.

Die erfindungsgemässen Verbindungen werden in Mengen von 0,005-1% oder mehr, bezogen auf das Gewicht des flüssigen oder pulverförmigen, fertigen Waschmittels, zugesetzt. Waschflotten, die die angegebenen Mengen der beanspruchten optischen Aufheller enthalten, verleihen beim Waschen von Textilien aus Cellulosefasern, Polyesterfasern, Wolle etc. einen brillanten Aspekt am Tageslicht.

Die Waschbehandlung wird beispielsweise wie folgt durchgeführt:

Die angegebenen Textilien werden während 1 bis 30 Minuten bei 20 bis 100° C in einem Waschbad behandelt, das 1 bis 10 g/kg eines aufgebauten, zusammengesetzten Waschmittels und 0,05 bis 1%, bezogen auf das Waschmittelgewicht, der beanspruchten Aufhellmittel enthält. Das Flottenverhältnis kann 1 : 3 bis 1 : 50 betragen. Nach dem Waschen wird wie üblich gespült und getrocknet. Das Waschbad kann als Bleichzusatz 0,2 g/l Aktivchlor (z.B. als Hypochlorid) oder 0,1 bis 2 g/l Natriumperborat enthalten.

In den folgenden Beispielen sind Prozent immer Gewichtsprozente; Schmelz- und Siedepunkte sind, sofern nicht anders vermerkt, unkorrigiert.

*Beispiele:*

*Beispiel 1a:*

39,1 g der Verbindung 13

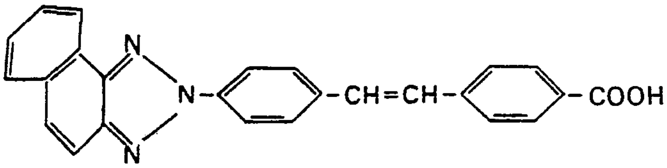

(13)

werden in 400 ml Xylol mit 1 g Dimethylformamid und 24 g Thionylchlorid 5 h unter Rückfluss gerührt, anschliessend das überschüssige Thionylchlorid abdestilliert, bei Raumtemperatur abgesaugt, mit Xylol und Hexan gewaschen und getrocknet. Man erhält 34,8 g (85% d.Th.) der Verbindung 14, welche ohne weitere Reinigung eingesetzt wird.

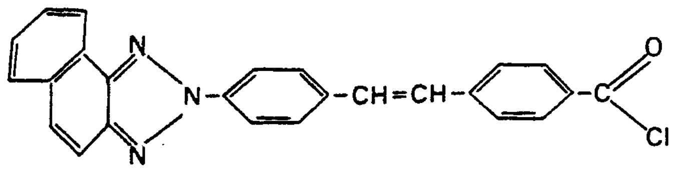

(14)

*Beispiel 1b:*

4,1 g des Säurechlorids 14 wurden in eine Lösung von 1 g Acetamidoxim in 40 ml DMF innerhalb 10 min eingetragen, 1 h bei 25°C und anschliessend 2 h am Rückfluss gerührt. Nach dem Abkühlen wird abgesaugt, mit Methanol gewaschen und getrocknet. Man erhält 3,1 g (73% d.Th.) der Verbindung 15

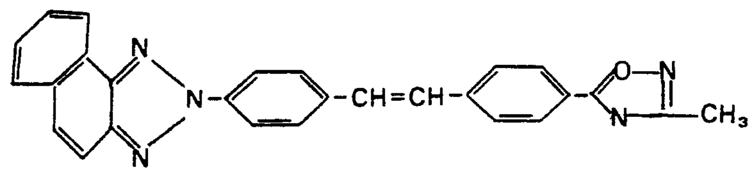

(15)

welche nach Umkristallisation aus DMF unter Klärung mit Aktivkohle bei 248°-250°C schmilzt.

Absorption (gem. in DMF):
λ max. = 377 nm ε = 7,10 × $10^4$

Fluoreszenz (gem. in DMF):
λ max. = 436 nm

*Beispiel 2:*

1 g Propionamidoxim wurden in 50 ml Toluol mit 1 g Triäthylamin vorgelegt und 4,1 g des Säurechlorids 14 eingetragen und 1 h nachgerührt. Es wird abgesaugt, mit Methanol und Wasser gewaschen und getrocknet. Man erhält 4,2 g der Verbindung 16

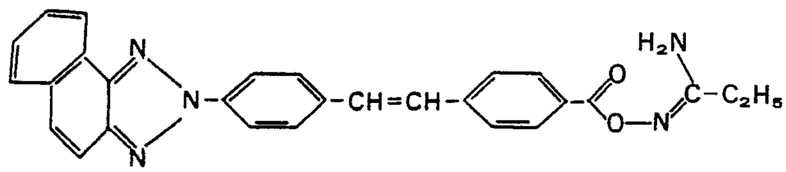

(16)

welche ohne weitere Reinigung in 40 ml N-Methylpyrrolidin 2 h bei 160°C gerührt wird. Nach dem Abkühlen wird abgesaugt, mit Methanol gewaschen und getrocknet. Man erhält 3,1 g (70% d.Th.) der Verbindung 17

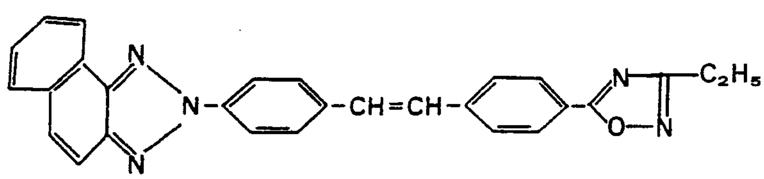

(17)

welche nach Umkristallisieren aus DMF unter Klärung mit Aktivkohle bei 219°-220°C schmilzt.

Absorption (gem. in DMF):
λ max. = 370 nm ε = 7,14 × $10^4$

Fluoreszenz (gem. in DMF):
λ max. = 437 nm

Analog Beispiel 2 erhält man die in nachfolgender Tabelle aufgeführten Verbindungen der Formel 18

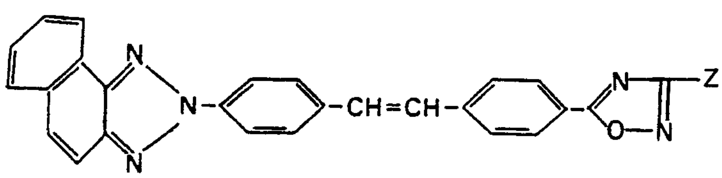

(18)

| Beispiel Nr. | Verb. Nr. | Z | Ausbeute (% d.Th.) | Fp[1] (°C) | Absorption | | Fluoreszenz (gem. in DMF) λ max. (nm) |
|---|---|---|---|---|---|---|---|
| | | | | | λ max. (nm) | $\times 10^4$ | |
| 3 | 19 | $-CH_2-CH_2-OH$ | 59 | 218-225 | 375 | 6,94 | 436 |
| 4 | 20 | $-CH_2-CH_2-O-CH_3$ | 69 | 187-197 | 375 | 6,92 | 437 |
| 5 | 21 | $-CH_2-C_6H_5$ | 54 | 258-259 | 376 | 7,34 | 438 |
| 6 | 22 | 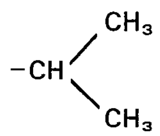 | 72 | 199-200 | 375 | 7,08 | 442 |
| 7 | 23 | 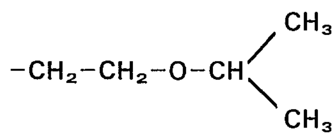 | 41 | 188-231 | | | |

[1] Die Verbindungen schmelzen teilweise unscharf.

**Patentansprüche für die Vertragsstaaten:** BE, CH, DE, FR, GB, IT, LI, NL, SE

1. Verbindungen der Formel 1

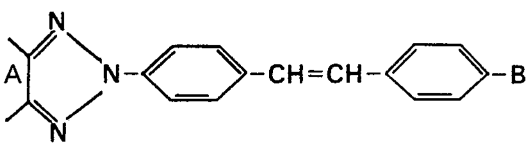 (1)

worin A einen gegebenenfalls durch nicht-chromophore Substituenten substituierten Benzol- oder Naphthalinring, B eine Gruppe der Formel

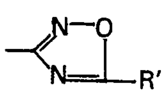 oder 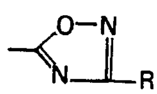

und R und R' ($C_1$ bis $C_6$)-Alkyl, ($C_1$ bis $C_6$)-Chloralkyl, ($C_1$ bis $C_4$)-Alkoxy-($C_1$ bis $C_4$)-Alkyl, Hydroxy-($C_1$ bis $C_4$)-Alkyl oder eine Gruppe der Formel $-(CH_2CH_2O)_n-R'$ bedeuten, wobei n 2 oder 3 und R' Wasserstoff oder ($C_1$ bis $C_4$)-Alkyl ist.

2. Verbindungen der Formel 1 nach Anspruch 1, wobei A eine Gruppe der Formel

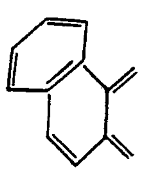

bedeutet und B für eine Gruppe der Formel

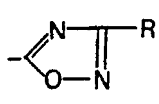

steht, in der R ($C_1$ bis $C_4$)-Alkyl, ($C_1$ bis $C_4$)-Chloralkyl, Hydroxy-($C_1$ bis $C_4$)-Alkyl oder ($C_1$ bis $C_4$)-Alkoxy-($C_1$ bis $C_4$)-Alkyl bedeutet.

3. Verbindungen der Formel 1 nach Anspruch 1, wobei A eine Gruppe der Formel

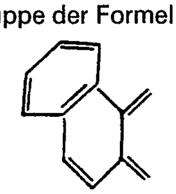

bedeutet und B für eine Gruppe der Formel

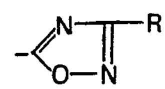

steht, in der R Methyl, Ethyl, i-Propyl, Hydroxyethyl oder Methoxyethyl bedeutet.

4. Verfahren zur Herstellung der Verbindungen der Formel 1 nach Anspruch 1, dadurch gekennzeichnet, dass man eine Verbindung der allgemeinen Formel 9

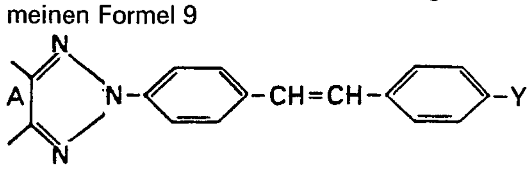 (9)

mit einer Verbindung der allgemeinen Formel 10

$$R-Z \quad (10)$$

umsetzt, wobei R die zuvor angegebenen Bedeutungen hat und Y eine Gruppe der Formel 11

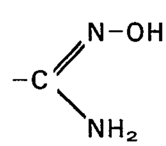 (11)

bedeutet, wobei Z gleichzeitig eine Gruppe der Formel 12

$$-COCl \quad (12)$$

darstellt oder Y bedeutet eine Gruppe der Formel 12 und Z ist gleichzeitig eine Gruppe der Formel 11.

5. Verwendung der Verbindungen nach Anspruch 1 als optische Aufheller.

**Patentansprüche für den Vertragsstaat:** AT

1. Verfahren zur Herstellung von Verbindungen der Formel 1

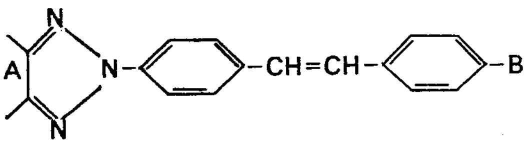 (1)

worin A einen gegebenenfalls durch nicht-chromophore Substituenten substituierten Benzol- oder Naphthalinring, B eine Gruppe der Formel

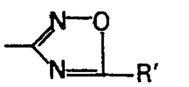 oder 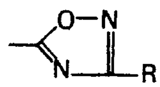

und R und R' ($C_1$ bis $C_6$)-Alkyl, ($C_1$ bis $C_6$)-Chloralkyl, ($C_1$ bis $C_4$)-Alkoxy-($C_1$ bis $C_4$)-Alkyl, Hydroxy-($C_1$ bis $C_4$)-Alkyl oder eine Gruppe der Formel $-(CH_2CH_2O)_n-R^1$ bedeuten, wobei n 2 oder 3 und $R^1$ Wasserstoff oder ($C_1$ bis $C_4$)-Alkyl ist, dadurch gekennzeichnet, dass man eine Verbindung der allgemeinen Formel 9

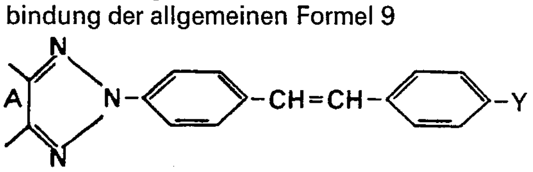 (9)

mit einer Verbindung der allgemeinen Formel 10

$$R-Z \quad (10)$$

umsetzt, wobei R die zuvor angegebenen Bedeutungen hat und Y eine Gruppe der Formel 11

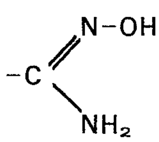 (11)

bedeutet, wobei Z gleichzeitig eine Gruppe der Formel 12

$$-COCl \quad (12)$$

darstellt oder Y bedeutet eine Gruppe der Formel 12 und Z ist gleichzeitig eine Gruppe der Formel 11.

2. Verfahren nach Anspruch 1, dadurch gekennzeichnet dass man Verbindungen der Formel 1 herstellt, wobei A eine Gruppe der Formel

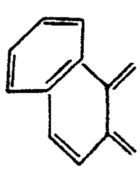

bedeutet und B für eine Gruppe der Formel

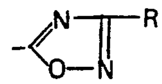

steht, in der R ($C_1$ bis $C_4$)-Alkyl, ($C_1$ bis $C_4$)-Chloralkyl, Hydroxy-($C_1$ bis $C_4$)-Alkyl oder ($C_1$ bis $C_4$)-Alkoxy-($C_1$ bis $C_4$)-Alkyl bedeutet.

3. Verfahren nach Anspruch 1, dadurch gekennzeichnet, dass man Verbindungen der Formel 1 herstellt, wobei A eine Gruppe der Formel

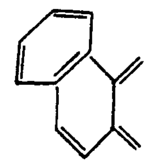

bedeutet und B für eine Gruppe der Formel

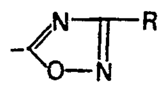

steht, in der R Methyl, Ethyl, i-Propyl, Hydroxyethyl oder Methoxyethyl bedeutet.

**Revendications pour les Etats contractants:** BE, CH, DE, FR, GB, IT, LI, NL, SE

1. Composés répondant à la formule 1

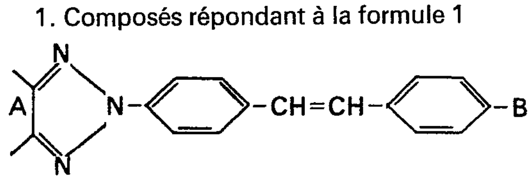 (1)

dans laquelle A représente un noyau benzénique ou napthalénique éventuellement porteur d'un substituant non chromophore et B représente un radical répondant à l'une des formules suivantes:

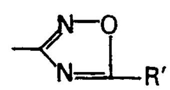 et 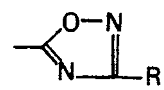

dans lesquelles R et R' représentent chacun un alkyle en $C_1$-$C_6$, un chloralkyle en $C_1$-$C_6$, un alcoxyalkyle dont chacune des parties alkyles contient de 1 à 4 atomes de carbone, un hydroxyalkyle en $C_1$-$C_4$ ou un radical $-(CH_2CH_2O)_n-R^1$ dans lequel n est égal à 2 ou à 3 et $R^1$ représente l'hydrogène ou un alkyle en $C_1$-$C_4$.

2. Composés de formule 1 selon la revendication 1 dans lesquels A représente un radical de formule:

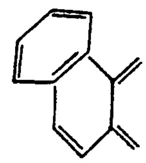

et B représente un radical répondant à la formule:

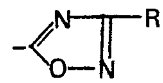

dans laquelle R représente un alkyle en $C_1$-$C_4$, un chloralkyle en $C_1$-$C_4$, un hydroxyalkyle en $C_1$-$C_4$ ou un alcoxyalkyle contenant de 1 à 4 atomes de carbone dans chacune de ses parties alkyles.

3. Composés de formule 1 selon la revendication 1 dans lesquels A représente un radical de formule

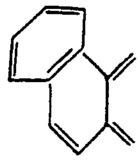

et B représente un radical répondant à la formule

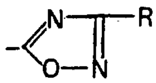

dans laquelle R représente un radical méthyle, éthyle, isopropyle, hydroxyéthyle ou méthoxyéthyle.

4. Procédé de préparation des composés de formule 1 selon la revendication 1, procédé caractérisé en ce qu'on fait réagir un composé de formule générale 9

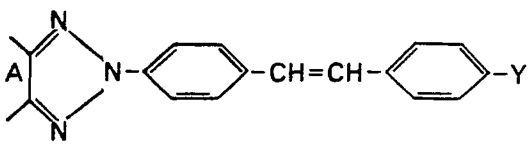 (9)

avec un composé de formule générale 10

R–Z (10)

formules dans lesquelles R a la signification précédemment donnée et Y représente un radical de formule 11

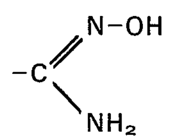 (11)

auquel cas Z représente un radical de formule 12

–COCl (12)

ou Y représente un radical de formule 12 et alors Z représente un radical de formule 11.

5. Application des composés selon la revendication 1 comme azureurs optiques.

**Revendications pour l'Etat contractant:** AT

1. Procédé de préparation de composés répondant à la formule 1

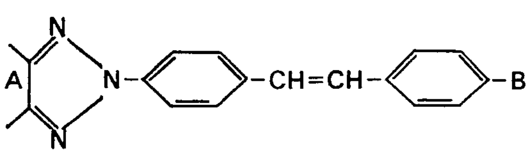 (1)

dans laquelle A représente un noyau benzénique ou naphtalénique éventuellement porteur d'un substituant non chromophore et B représente un radical répondant à l'une des formules suivantes:

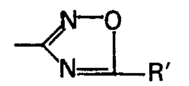 et 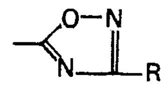

dans lesquelles R et R' représentent chacun un alkyle en $C_1$-$C_6$, un chloralkyle en $C_1$-$C_6$, un alcoxyalkyle dont chacune des parties alkyles contient de 1 à 4 atomes de carbone, un hydroxyalkyle en $C_1$-$C_4$ ou un radical $-(CH_2CH_2O)_n-R^1$ dans lequel n est égal à 2 ou à 3 et $R^1$ représente l'hydrogène ou un alkyle en $C_1$-$C_4$, procédé caractérisé en ce qu'on fait réagir un composé de formule générale 9

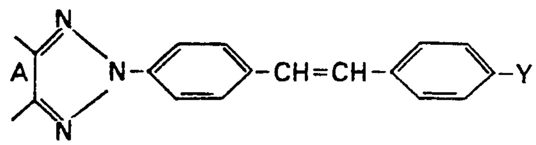 (9)

avec un composé de formule générale 10

R–Z (10)

formules dans lesquelles R a la signification précédemment donnée et Y représente un radical de formule 11

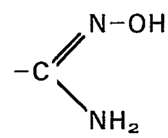 (11)

auquel cas Z représente un radical de formule 12

–COCl (12)

ou Y représente un radical de formule 12 et alors Z représente un radical de formule 11.

2. Procédé selon la revendication 1 caractérisé en ce que l'on prépare des composés de formule 1 dans lesquels A représente un radical de formule

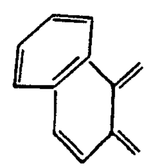

et B représente un radical répondant à la formule:

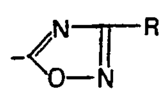

dans laquelle R représente un alkyle en $C_1$-$C_4$, un chloralkyle en $C_1$-$C_4$, un hydroxyalkyle en $C_1$-$C_4$ ou un alcoxyalkyle contenant de 1 à 4 atomes de carbone dans chacune de ses parties alkyles.

3. Procédé selon la revendication 1 caractérisé en ce qu'on prépare des composés de formule 1 dans lesquels A représente un radical de formule

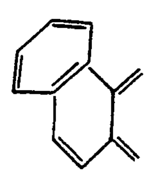

et B représente un radical répondant à la formule

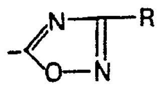

dans laquelle R représente un radical méthyle, éthyle, isopropyle, hydroxyéthyle ou méthoxyéthyle.

**Claims for the Contracting States:** BE, CH, DE, FR, GB, IT, LI, NL, SE

1. Compounds of the formula 1

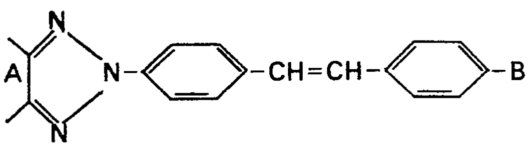 (1)

wherein A is a benzene or naphthalene ring, optionally substituted by non-chromophoric substituents, B is a group of the formulae

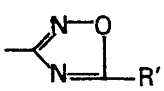 or 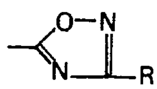

and R and R' mean $(C_1-C_6)$-alkyl, $(C_1-C_6)$-chloroalkyl, $(C_1-C_4)$-alkoxy-$(C_1-C_4)$-alkyl, hydroxy-$(C_1-C_4)$-alkyl or a group of the formula $-(CH_2CH_2O)_n-R'$, wherein n is 2 or 3 and R' is a hydrogen atom or $(C_1-C_4)$-alkyl.

2. Compounds of the formula 1, according to claim 1, wherein A means a group of the formula

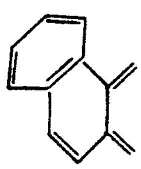

and B is a group of the formula

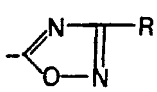

wherein R is $(C_1-C_4)$-alkyl, $(C_1-C_4)$-chloralkyl, hydroxy-$(C_1-C_4)$-alkyl or $(C_1-C_4)$-alkoxy-$(C_1-C_4)$-alkyl.

3. Compounds of the formula 1, according to claim 1, wherein A is a group of the formula

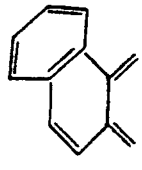

and B means a group of the formula

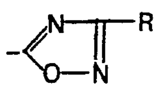

wherein R means methyl, ethyl, i-propyl, hydroxyethyl or methoxyethyl.

4. Process for the manufacture of the compounds of the formula 1, according to claim 1, which comprises reacting a compound of the general formula 9

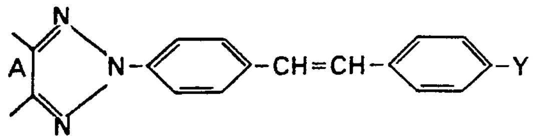 (9)

with a compound of the general formula 10

$$R-Z \qquad (10)$$

wherein R has the afore-mentioned meanings and Y is a group of the formula 11

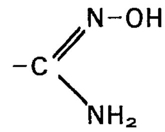 (11)

Z, being a group of the formula 12

$$-COCl \qquad (12)$$

or Y means a group of the formula 12 and Z is a group of the formula 11.

5. Method of use of the compounds as claimed in claim 1 as optical brighteners.

**Claims for the Contracting State:** AT

1. Process for the manufacture of the compounds of the formula 1

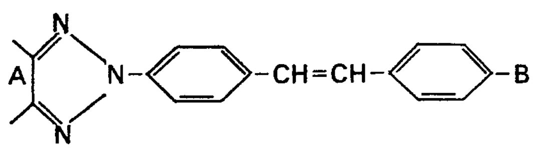 (1)

wherein A is a benzene or naphthalene ring, optionally substituted by non-chromophoric substituents, B is a group of the formulae

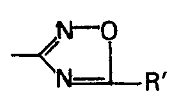 or 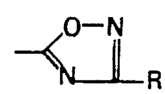

and R and R' mean $(C_1-C_6)$-alkyl, $(C_1-C_6)$-chloroalkyl, $(C_1-C_4)$-alkoxy-$(C_1-C_4)$-alkyl, hydroxy-$(C_1-C_4)$-alkyl or a group of the formula $-(CH_2CH_2O)_n-R^1$, wherein n is 2 or 3 and $R^1$, is a hydrogen atom or $(C_1-C_4)$-alkyl, which comprises reacting a compound of the general formula 9

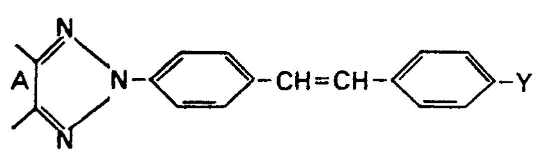 (9)

with a compound of the general formula 10

$$R-Z \qquad (10)$$

wherein R has the afore-mentioned meanings and Y is a group of the formula 11

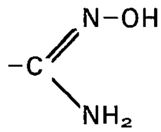

(11)

Z, being a group of the formula 12

$$-COCl \qquad (12)$$

or Y means a group of the formula 12 and Z is a group of the formula 11.

2. Process as claimed in claim 1, which comprises preparing a compound of formula 1 wherein A means a group of the formula

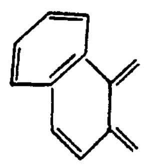

and B is a group of the formula

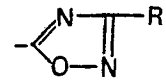

wherein R is ($C_1$-$C_4$)-alkyl, ($C_1$-$C_4$)-chloroalkyl, hydroxy-($C_1$-$C_4$)-alkyl or ($C_1$-$C_4$)-alkoxy-($C_1$-$C_4$)-alkyl.

3. Process as claimed in claim 1, which comprises preparing a compound of formula 1, wherein A is a group of the formula

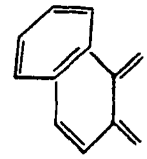

and B means a group of the formula

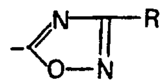

wherein R means methyl, ethyl, i-propyl, hydroxyethyl or methoxyethyl.